## Europäisches Patentamt

## European Patent Office

## Office européen des brevets

(11) Publication number: **0 160 562**
**B1**

# EUROPEAN PATENT SPECIFICATION

(45) Date of publication of patent specification: **28.03.90**

(21) Application number: **85303027.8**

(22) Date of filing: **29.04.85**

(51) Int. Cl.⁵: **C 07 C 211/00**, C 07 C 209/00 // C07C215/00

(54) Process for the continuous preparation of P-phenylenediamine from a nitrobenzene feedstock.

(30) Priority: **30.04.84 US 605437**

(43) Date of publication of application:
**06.11.85 Bulletin 85/45**

(45) Publication of the grant of the patent:
**28.03.90 Bulletin 90/13**

(84) Designated Contracting States:
**BE DE FR GB IT NL**

(56) References cited:
**US-A-3 383 416**
**US-A-4 256 669**
**US-A-4 400 537**

**Patent Abstracts of Japan, unexamined applications, section C, vol. 2, no. 8, Jan. 20, 1978**

**THE PATENT OFFICE JAPANESE GOVERMENT, page 3737 C 77**

**Patent Abstracts of Japan, unexamined applications. field C, vol. 5, no. 204, Dez. 24, 1981**

(73) Proprietor: **Montvale Process Company Incorporated**
**190 Sherman Street**
**Fairfield Connecticut 06430 (US)**

(72) Inventor: **Costa, Lawrence C.**
**3 Lisa lane**
**Manuet New York 10954 (US)**
Inventor: **Wan, Chee-Gen**
**17 Warwick Avenue**
**Fort Lee New York 07024 (US)**
Inventor: **Porcelli, Richard V.**
**287 Crestwood Avenue**
**Yonkers New York 10707 (US)**

(74) Representative: **Ritter, Stephen David et al**
**Mathys & Squire 10 Fleet Street**
**London EC4Y 1AY (GB)**

(56) References cited:
**THE PATENT OFFICE JAPANESE GOVERMENT, page 104 C 85**

Courier Press, Leamington Spa, England.

## Description

Background of the invention
Field of the invention

This invention relates to an improved method for the preparation of aromatic diamines from a nitrobenzene feedstock, and more particularly relates to the continuous preparation of p-phenylenediamine (PPD) through the amination of a p-aminophenol (PAP) intermediate which is substantially recovered due to the utilization of an effective water immiscible, basic, organic solvent.

Description of the prior art

P-aminophenol (PAP) is a well known and highly useful industrial chemical which is primarily used as an intermediate for the production of dyestuffs and photographic chemicals and as an intermediate in the production of pharmaceuticals.

P-phenylenediamine (PPD) is a valuable industrial chemical which is used primarily as an an antioxidant and as an intermediate in the manufacture of aramids. PPD is usually made commercially by the catalytic hydrogenation of p-nitroaniline.

A wide variety of methods for the preparation and subsequent purification of PAP are known to those in the art, particularly the hydrogenation of nitrobenzene in an aqueous acidic medium as is disclosed in U.S. Patents 3,383,416, 3,694,508, 3,717,680, 3,953,509, 4,224,247, 4,264,529 and 4,307,249.

U.S. Patent 3,383,416 discloses a particularly useful process involving the catalytic hydrogenation of nitrobenzene in an aqueous sulfuric acid medium. In accordance with this process, the reduction of the nitrobenzene is interrupted prior to completion at a point at which the reaction mixture still comprises a sufficient amount of unreacted nitrobenzene and forms an immiscible layer of nitrobenzene which also contains the precious metal hydrogenation catalyst suspended therein. A second aqueous layer contains a p-aminophenol sulfate product. The aqueous layer is separated from the nitrobenzene layer and the PAP is eventually isolated; the nitrobenzene layer containing the hydrogenation catalyst is recycled to the hydrogenation zone.

A serious flaw of the aforementioned catalytic hydrogenation process is that the subsequent isolation and purification of solid PAP from the crude reaction mixture is extremely cumbersome and expensive, and the various isolation and purification schemes which have been disclosed in U.S. Patents 3,658,905, 3,694,508, 3,876,703, 4,139,562 and 4,224,247 are not readily amenable to continuous processing.

The amination of PAP to produce PPD is a lesser known reaction, although it has been investigated, particularly by Japanese workers. Japanese patents 7414737 and 7414738 disclose the amination of phenols carried out in the presence of γ-alumina which has been treated with either alkali, carboxylic or boric acids in the presence of a molar excess of ammonia at temperatures of about 365°C to produce PPD and similar compound. Canadian patent 1,006,183 discloses treating PAP with aqueous ammonia in the presence of several palladium catalysts at conditions in excess of 200°C and 4.8—5.9 MPa (700—850 psig) in the presence of hydrogen. Japanese Patent 7723030 discloses the reaction of PAP with aqueous ammonia in the presence of a hydrated cobalt catalyst at elevated temperatures and pressures to produce PPD. Japanese Patent 7742829 discloses the reaction of hydroquinone with aqueous ammonia in the presence of the same cobalt catalyst under similar conditions to produce a mixture of PAP and PPD. Japanese Patent 80153751 discloses the reaction of PAP with a large excess of ammonia in the presence of tin chloride and a phenol solvent to produce PPD in moderate conversions and selectivities.

Japanese Patent 77108933 (Mitsui Petrochemicals Inc.) discloses a reaction mixture of purified PAP and a molar excess of ammonia passed over a solid acid catalyst at temperatures of 280—400°C to produce PPD. This process however, does not describe an effective and economical method of preparing a PAP feedstock, and also does not utilize an effective solvent.

U.S. Patent 4,400,537 describes the amination of either hydroquinone or PAP to PPD in the presence of an alumina catalyst, at temperatures of 325—450°C, a high molar ratio of ammonia to reactant, and an inert, liquid hydrocarbon solvent with a boiling point of from 25—400°C. The usage of such a solvent is not predicated on improving the recovery of PAP from the reaction mixture, but is instead utilized to improve the later amination reaction to PPD.

U.S. Patent 3,860,650 discloses a process and accompanying alumina catalyst for the production of organic amines by the amination of phenolic type compounds.

It is, therefore, an object of the present invention to provide an effective and economic method for the continuous preparation of p-phenylenediamine (PPD) from a nitrobenzene feedstock.

It is another object of the invention to provide an effective and economic method of recovering the PAP intermediate for subsequent amination as the PPD feedstock.

It is still another object of the invention to provide a method which produces high yields of PPD from a nitrobenzene feedstock, through a PAP intermediate.

Summary

In accordance with the aforementioned objects, a novel process is disclosed for the efficient, continuous preparation of p-phenylenediamine (PPD) from a nitrobenzene feedstock through the formation and amination of a p-aminophenol (PAP) intermediate; the process comprising reacting nitrobenzene with hydrogen and aqueous sulfuric acid in a reduction zone to form PAP-sulfate in the presence of an effective hydrogenation catalyst; separating a formed immiscible nitrobenzene phase containing the hydrogenation catalyst from a formed aqueous phase containing the product PAP-sulfate, other unused reactants and byproducts; preferably recycling the nitrobenzene phase to the reaction zone; neutralizing the aqueous PAP-sulfate containing phase with an effective base, e.g. ammonia; dissolving and isolating the formed PAP, and other reaction products, in a water immiscible, basic, organic solvent, e.g. aniline; and transporting the PAP-containing solvent solution to an amination zone, aminating the PAP containing solvent solution with a molar excess of ammonia, to convert PAP contained therein to PPD at temperatures ranging between 200—400°C and pressures between 345—6890 KPa (50—1000 psia), preferably in the presence of an acidic alumina catalyst; separating the formed PPD from the basic organic solvent-reaction byproduct mixture, preferably by crystallization or distillation.

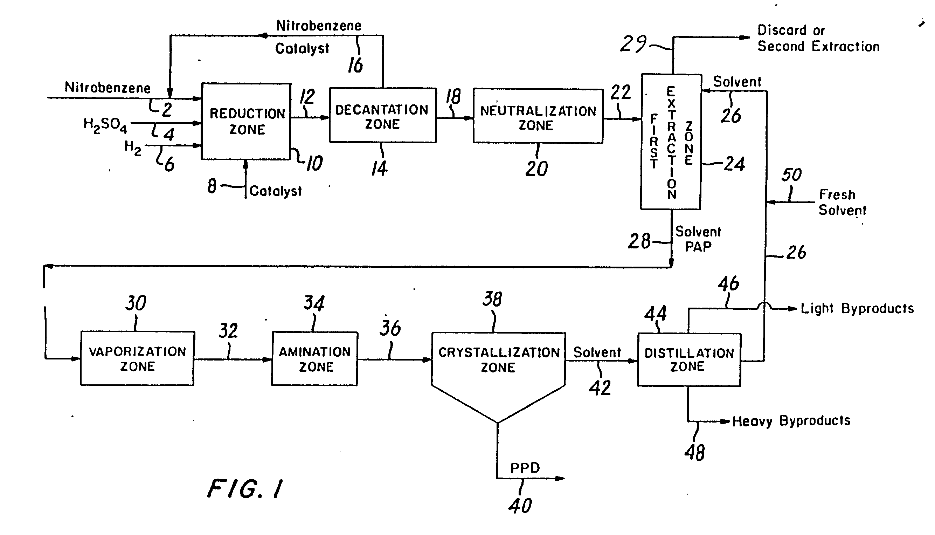

Figure 1 discloses a simplified outline of the preferred process of the invention.

## Detailed description of the invention

The method of the present invention is based upon a process for converting a nitrobenzene feedstock into p-phenylenediamine (PPD), with the reaction proceeding through a p-aminophenol (PAP) intermediate; the PAP intermediate being substantially and economically recovered from the reaction mixture by contacting with a member of a class of basic organic solvents, and various mixtures thereof, each solvent being substantially immiscible with water and capable of dissolving the solid PAP at temperatures of 20 to 200°C. Surprisingly, we have discovered that PAP can be more easily recovered from the crude admixture and conveyed to the amination reaction zone by contacting the formed reaction mixture with members of a class of solvents which will dissolve PAP, in contrast to U.S. 4,224,247 in which an organic amine is used to suspend the PAP which may subsequently be separated from the solvent-PAP mixture by filtration, if desired.

The presence of a basic organic solvent or solvent mixture is required because of the processing problems caused by the limited solubility of PAP in most of the common organic solvents, thereby rendering continuous product separation operations unsuitable. The solvent must also be capable of withstanding elevated temperatures as high as about 400°C when in the presence of ammonia acting as amination agent and accompanying catalyst. Many aliphatic solvents which might otherwise be suitable will decompose when exposed to such process conditions. However, a class of water immiscible basic, organic solvents including aniline, quinoline, benzonitrile, isomeric toluidines, xylidines, picolines, lutidines, quinaldine, various mixtures thereof and the like have surprisingly been found to be particularly adaptable for use in the process. Aniline is particularly adaptable for use in the process. Aniline is particularly preferred since it is also a byproduct of both stages of the integrated process.

The amount of solvent employed in the process cycle depends upon a variety of parameters, such as the precise solvent or solvent mixture involved, the temperatures of the process operation, the amount of impurities and byproducts present in the crude reaction mixtures, etc. In general the higher the overall temperature of the process the smaller the amount of solvent required, since solvents usually have a greater solvating power at elevated temperatures. In addition, the prefered solvent will also display differing degrees of solvating power for many of the different constituents present in the crude reaction mixtures.

Although the particular solvent of choice can be contacted with the PAP containing reaction mixture in a variety of ways, it is preferred to either admix the solvent with an aqueous suspension of the crude PAP or preferably, the aqueous, neutralized suspension obtained through the catalytic hydrogenation of nitrobenzene in aqueous sulfuric acid as set forth in U.S. Patent No. 3,383,416.

The mole % of nitrobenzene feedstock eventually converted to PPD in the process can range from as littLe as about 20 mole % of the nitrobenzene to as much as 90 mole %. In the most preferred embodiment the reaction will consume about 1.3 moles of nitrobenzene per mole of PPD. The major byproduct derived from nitrobenzene in the reaction is aniline.

As can be seen in figure 1, the aqueous acidic solution of PAP is initially prepared by reacting nitrobenzene 2, sulfuric acid 4 and hydrogen 6 feedstreams in the presence of a suitable hydrogenation catalyst 8, preferably platinum or palladium on carbon, in a reduction zone 10 under typical reaction conditions as are set forth in U.S. Patent 3,383,416. A reaction product containing the PAP sulfate salt, aniline sulfate, along with various other byproducts and reactants passes from the reduction zone through line 12 and enters a decantation zone 14, where it is separated into two substantially immiscible phases, with the phase containing nitrobenzene and the hydrogenation catalyst being recycled back through line 16 to the reduction zone 10 for further usage, while the aqueous phase which contains the PAP sulfate, and other reactant products is passed through line 18 to a neutralization zone 20, in which the mixture is contacted with an effective base, preferably ammonia, of effective strength to neutralize the acidic reaction mixture. Despite the fact that nitrobenzene is relatively inexpensive, the cost of producing PAP by prior art methods is extremely high, due to the labor and equipment necessitated by the many processing steps, e.g., crystallization, filtration, purification, etc. involved in separating solid PAP from the reaction mixture, accompanied by the inevitable losses of product which occur during each operation.

The PAP containing suspension, together with any accompanying byproducts and impurities, is passed to a first extraction zone 24 through line 22, with the mixture maintained at a temperature of about 20 to 100°C, and contacted with the water immiscible, basic, organic solvent stream 26 as described earlier. Upon completion of the extraction, a first stream 28 primarily comprising the solvent transporting the dissolved PAP exits the extraction zone, which may consist of more than one separation unit, is first concentrated and then passed into the amination zone, while a second stream 29 containing $(NH_4)_2SO_4$, water, some solvent, PAP and various byproducts is removed from the system and either discarded, or, further separated if so desired.

The solvent-carrying PAP stream may also include some of the still remaining lighter organics as it enters a vaporization zone 30 in which the stream is heated to about 200 to 400°C and is contacted with ammonia as amination agent.

The amount of amination reagent used per mole of PAP can range from about 1 to 500 moles, and preferably about 20 to 200 moles.

The PAP/solvent mixture is then passed through line 32 into an amination zone 34 maintained at a temperature of about 200 to 400°C and at a pressure of about 50 to 1000 psia, preferably at temperatures of about 300 to 380°C and pressures of about 50 to 500 psia. Once in the zone, the PAP, ammonia and solvent containing stream are contacted with a suitable alumina catalyst and react to form PPD. The amount of PAP present in the amination zone can range from about 5 to 75 wt. % of solvent present, most preferably about 10 to 50 wt. %.

The term "alumina" covers a wide variety of specific entities which have varying physico-chemical properties and the matter is further complicated by the existence of different crystalline forms having essentially the same gross chemical composition. Further, in addition to the three more or less well defined degrees of hydration, there exists a number of "transition aluminas", identifiable by crystallographic and other physico-chemical criteria. The matter is still further complicated by the intermingling of two or more systems of nomenclature. To illustrate, one crystalline entity having the nominal composition of $Al(OH)_3$ is known variously as alpha-alumina trihydrate, gibbsite and hydrargyllite. Similarly, one crystalline entity having the nominal composition represented at AlOOH is known as alpha-alumina oxide hydroxide, alpha-alumina monohydrate or boehmite. The end product of thermal degradation of all the forms of hydrated alumina is $Al_2O_3$, known as alpha-alumina or corundum. The term "activated alumina" has been applied broadly to the transition aluminas. The whole matter of the interrelationships of the various types of "alumina" is discussed in the Kirk-Othmer "Encyclopedia of Chemical Technology", 3rd Ed. Vol. 2, pp, 218—244 (John Wiley & Sons, New York) 1978, which is incorporated here by reference.

The preferred alumina catalysts for the process can comprise alumina, alumina-magnesia, alumina-boria, alumina-silica, alumina-titania and the like. The most preferred alumina for the practice of the process is sold under the trade name "H151" by Alcoa, Inc., and prepared according to the teachings of U.S. Patent 3,860,650.

Upon completion of the amination reaction the effluent stream 36 containing the product PPD, along with the remaining unreacted reactants and the reaction byproducts, is passed to a suitable separation zone. Preferably a crystallization unit, e.g., zone 38 is included therein, and the PPD crystals are removed from the solvent, reactant and byproduct containing stream, e.g., line 40.

The exiting stream 42 is then passed into a distillation zone 44 for removal of the light (line 46) and heavy (line 48) byproducts that would otherwise build up as the bulk of the solvent stream is recycled back through line 26 to the PAP section. It is preferred to replenish the solvent stream by a fresh amount of solvent from line 50 for effective continuous operation.

The above discussion demonstrate that the disclosed invention has the advantage of providing a low cost PAP feedstock, transported by an effective solvent, which can be directly aminated in the presence of an alumina catalyst to produce the desired PPD product. Such a process surprisingly eliminates the need for expensive intermediate separation and purification operations, thereby greatly improving process economics.

It will be understood that, although the crude PAP solution utilized in the process of this invention is preferably derived from the reduction of nitrobenzene with hydrogen in an acid solution, e.g., a sulfuric acid solution as described in U.S. Patent 3,383,416, crude p-aminophenol derived from other processes may also be utilized by introducing such material into a suitable solvent or solvent mixture and proceeding accordingly.

The following examples are provided to illustrate the invention in accordance with the principles of this invention but are not construed as limiting the invention in any way except as indicated by the appended claims.

Example 1

This example demonstrates the conversion of nitrobenzene to p-aminophenol according to the procedure set forth in U.S. Patent 3,383,416. A 500 mL 3-neck Morton flask was charged with 170 mL of deionized water, 0.15 g 5% platinum on carbon, and 0.10 g "Aliquat 336" (a registered trademark of Henkel Corp). which is a mixture of tetraalkylammonium chlorides. The flask was purged with nitrogen and the mixture was heated with mechanical stirring to 65°C. At this point, 20.7 g of sulfuric acid and 22.5 mL of nitrobenzene were cautiously added to the flask. The resulting mixture was heated to 85—90°C, the nitrogen purge was stopped, and hydrogen was sparged through this mechanically stirred mixture for 5 hours while the temperature was maintained at 85—90°C. The hydrogen back-pressure in the system was maintained by a bubbler tube filled with a 1 cm column of mercury. At the end of the 5 hour reaction period, the stirring was stopped, the hydrogen sparge was replaced with a nitrogen purge, and the mixture was allowed to cool and separate into 2 phases. The 5% platinum on carbon hydrogenation catalyst remained suspended in 1.5 g of unreacted nitrobenzene. The clear aqueous phase was recovered by decantation, and the pH was adjusted to about 8 by addition of sufficient 29% aqueous ammonia. The precipitated p-aminophenol was collected by filtration, washed with water and a small amount of diethyl ether, and dried. The crude PAP weighed 17.4 g, and melted at 185—189°C. The yield of crude PAP based on consumed nitrobenzene was 78%. Also obtained was 4.5 g of byproduct aniline.

Example 2

The aqueous reaction mixture obtained in Example 1 was simulated by charging 5.0 g p-aminophenol, 50 mL water, 0.03 g "Aliquat 336", 1.3 g aniline, and 6.5 g sulfuric acid into a 250 mL 3-neck round bottom flask. This mixture became homogeneous on stirring and heating to 85°C under a nitrogen atmosphere. This mixture was cautiously neutralized by the slow addition of 8.5 g of 29% aqueous ammonia at 85°C. A precipitate of p-aminophenol was observed, and to this mixture was added 50 mL of aniline. The resulting mixture was stirred for 5 minutes at 85°C, the stirring was stopped, and the mixture separated into 2 homogeneous phases. No solids were observed. The upper aniline layer was removed by cannula and weighed 55.8 g. Some PAP precipitated from this aniline solution on cooling to room temperature, and this mixture was homogenized by the addition of 20 mL of pyridine. Analysis of the recovered, homogenized organic phase showed it to contain 3.1 g of PAP, for a single stage recovery of 62%.

Example 3

A 36 inch by 1.25 inch i.d. tubular reactor was charged with 125 cc of "H151" alumina catalyst prepared according to the teachings of U.S. Patent 3,860,650. The remaining volume was filled with glass spheres, and the reactor was electrically heated to 370°C. Liquid ammonia and a solution of 10% p-aminophenol, 20% aniline, and 70% pyridine (by weight) were simultaneously fed to the reactor. The internal pressure was maintained at 300 psig. After 36 hours of operation, 2,963.4 g of ammonia, 105.3 g of PAP, and 210.6 g of aniline had been fed to the reactor. The effluent was found to contain 1.2 g of PAP and 85.5 g of p-phenylenediamine. The PAP conversion was 99% and the selectivity to p-phenylenediamine was 83%.

Example 4

The procedure of Example 2 was repeated except that 40 mL of quinoline was used in place of 50 mL of aniline, and the extraction was performed at 80°C. The recovered quinoline phase was found to contain 3.8 g of PAP, for a single stage recovery of 76%.

Example 5

The procedure of Example 3 was repeated, except that the reactor pressure was maintained at 700 psi, and the weight ratio of aniline to PAP in the feed mixture was adjusted to 4:1. After 42 hours of continuous operation, 3161.4 g of ammonia, 123.6 g of PAP, and 494.4 g of aniline had been fed to the reactor. The effluent was found to contain 0.3 g PAP and 92.4 g p-phenylenediamine. The PAP conversion was over 99% and the selectivity to p-phenylenediamine was 76%.

Example 6

The procedure of Example 3 was repeated except that the reactor temperature was set at 360°C, the pressure was 200 psi, and the PAP was fed to the reactor as a 10% (by weight) solution in quinoline. After 35 hours of continuous operation, 1975.2 g of ammonia and 86.1 g of PAP had been fed to the reactor. The effluent was found to contain 16.9 g of PAP, 52.6 g of p-phenylenediamine, and 3.9 g of aniline. The PAP conversion was 80%, the selectivity to p-phenylenediamine was 77%, and the selectivity to aniline was 7%.

**Claims**

1. A process for preparing p-phenylenediamine (PPD) from a nitrobenzene feedstock comprising:

reacting nitrobenzene with hydrogen and aqueous sulfuric acid in a reduction zone to form p-aminophenol sulfate (PAP-sulfate) in the presence of an effective hydrogenation catalyst;

separating a formed immiscible nitrobenzene phase containing the hydrogenation catalyst from a formed aqueous phase containing the product (PAP) sulfate, other unused reactants and byproducts;

neutralizing the aqueous PAP-sulfate containing phase with an effective base;

dissolving and isolating the formed PAP in a water immiscible, basic, organic solvent and transporting the PAP solvent solution to an amination zone;

aminating the PAP containing solvent solution with a molar excess of ammonia to convert PAP contained therein to PPD at temperatures ranging between 200—400°C and pressures between 345—6890 KPa (50—1000 psia);

separating the formed PPD from the basic organic solvent-reaction byproduct mixture.

2. A process as claimed in claim 1 wherein the hydrogenation catalyst is selected from platinum on carbon and palladium on carbon.

3. A process as claimed in claim 1 or claim 2 wherein the immiscible unreacted nitrobenzene phase containing the hydrogenation catalyst is recycled to the reduction zone.

4. A process as claimed in any preceding claim wherein the effective base neutralizing the aqueous PAP-sulfate containing phase is ammonia.

5. A process as claimed in any preceding claim wherein the PAP containing solution is contacted and mixed with the water immiscible, basic, organic solvent in an extraction zone.

6. A process as claimed in claim 5, wherein the resultant mixture is separated into a first stream comprising the solvent transporting the dissolved PAP and a second stream tranporting a variety of other reaction products, and some solvent.

7. A process as claimed in any preceding claim wherein the water immiscible, basic, organic solvent is selected from aniline, quinoline, benzonitrile, toluidine, xylidine, picoline, lutidine, quinaldine and mixtures thereof.

8. A process as claimed in claim 7 wherein the solvent is aniline.

9. A process as claimed in any preceding claim wherein the amination zone is maintained at temperatures of 300—380°C and pressures of 345—3450 KPa (50—500 psia).

10. A process as claimed in any preceding claim wherein the amount of PAP in the amination zone ranges from 10 to 50 wt. % of the solvent present.

11. A process as claimed in any preceding claim wherein the amination is carried out in the presence of an acidic alumina catalyst.

12. A process as claimed in any preceding claim wherein the PPD product is removed from the reaction mixture in a separation zone by crystallisation.

13. A process as claimed in any preceding claim wherein the process is carried out continuously.

14. A process as claimed in any preceding claim wherein the solvent is recycled back to contact a fresh PAP containing solution in the extraction zone.

**Patentansprüche**

1. Verfahren zur Herstellung von p-Phenylendiamin (PPD) aus einer Nitrobenzol enthaltenden Beschickung,

dadurch gekennzeichnet, daß Nitrobenzol mit Wasserstoff und wäßriger Schwefelsäure in einer Reduktionszone in Gegenwart eines wirksam Hydrierungskatalysators unter Bildung von p-Aminophenolsulfat (PAP-Sulfat) umgesetzt wird,

eine gebildete nicht mischbare Nitrobenzolphase, die den Hydrierungskatalysator enthält, von einer gebildeten wäßrigen Phase, die das gewünschte Produkt (PAP) als Sulfat, andere nicht gebrauchte Reaktanten und Nebenprodukte enthält, angetrennt wird,

die PAP-Sulfat enthaltende wäßrige Phase mit einer wirksamen Base neutralisiert wird,

das gebildete PAP in einem mit Wasser nicht mischbaren basischen organischen Lösungsmittel gelöst und isoliert und die PAP-Lösung zu einer Aminierungszone transportiert wird,

die das PAP enthaltende Lösung mit einem molaren Überschuß an Ammoniak bei Temperaturen im Bereich von 200°C bis 400°C und Drükken zwischen 345 und 6890 kPa (50 bis 1000 psia) zwecks Umwandlung des in der Lösung enthaltenen PAP zu PPD aminiert wird und

das gebildete PPD vom Gemisch aus basischem organischem Lösungsmittel und Reaktionsnebenprodukten abgetrennt wird.

2. Verfahren nach Anspruch 1, dadurch gekennzeichnet, daß als Hydrierungskatalysator ein Platin-auf-Kohle-Katalysator und/oder ein Palladium-auf-Kohle-Katalysator verwendet wird.

3. Verfahren nach Anspruch 1 oder 2, dadurch gekennzeichnet, daß die den Hydrierungskatalysator enthaltende nicht mischbare und nicht umgesetzte Nitrobenzolphase in die Reduktionszone rückgeführt wird.

4. Verfahren nach einem der vorhergehenden Ansprüche, dadurch gekennzeichnet, daß als wirksame Base zur Neutralisation der PAP-Sulfat enthaltenden wäßrigen Phase Ammoniak verwendet wird.

5. Verfahren nach einem der vorhergehenden Ansprüche, dadurch gekennzeichnet, daß die PAP enthaltende Lösung in einer Extraktionszone mit dem mit Wasser nicht mischbaren basischen organischen Lösungsmittel zusammengebracht und vermischt wird.

6. Verfahren nach Anspruch 5, dadurch gekennzeichnet, daß das erhaltene Gemisch in einen ersten Strom, der das das gelöste PAP transportierende Lösungsmittel enthält, und in einen zweiten Sprom, der eine Reihe anderer Reaktionsprodukte und etwas Lösungsmittel enthält, aufgetrennt wird.

7. Verfahren nach einem der vorhergehenden Ansprüche, dadurch gekennzeichnet, daß das mit Wasser nichtmischbare basische organische Lösungsmitel aus Anilin, Chinolin, Benzonitril, Toluidin, Xylidin, Picolin, Lutidin, Chinaldin und Gemischen hiervon ausgewählt wird.

8. Verfahren nach Anspruch 7, dadurch gekennzeichnet, daß das Lösungsmittel Anilin ist.

9. Verfahren nach einem der vorhergehenden Ansprüche, dadurch gekennzeichnet, daß die Aminierungszone auf einer Temperatur von 300°C bis 380°C und auf Drücken von 345 kPa bis 3450 kPa (50 bis 500 psia) gehalten wird.

10. Verfahren nach einem der vorhergehenden Ansprüche, dadurch gekennzeichnet, daß die Menge an PAP in der Aminierungszone von 10 bis 50 Gewichtsprozent des vorhandenen Lösungsmittels reicht.

11. Verfahren nach einem der vorhergehenden Ansprüche, dadurch gekennzeichnet, daß die Aminierung in Anwesenheit eines sauren Aluminiumoxidkatalysators durchgeführt wird.

12. Verfahren nach einem der vorgergehenden Ansprüche, dadurch gekennzeichnet, daß das als Produkt gebildete PPD in einer Abtrennzone durch Kristallisation vom Reaktionsgemisch entfernt wird.

13. Verfahren nach einem der vorhergehenden Ansprüche, dadurch gekennzeichnet, daß das Verfahren kontinuierlich durchgeführt wird.

14. Verfahren nach einem der vorhergehenden Ansprüche, dadurch gekennzeichnet, daß das Lösungsmittel zwecks Kontakt mit einer frischen PAP enthaltenden Lösung in die Extraktionszone rückgeführt wird.

## Revendications

1. Procédé pour préparer de la p-phénylène-diamine (PPD) à partir d'une charge de nitrobenzène d'alimentation, comprenant:

la réaction du nitrobenzène avec de l'hydrogène et de l'acide sulfurique aqueux, dans une zone de réduction, pour former du sulfate de p-aminophénol (sulfate de PAP) en présence d'un catalyseur d'hydrogénation efficace;

la séparation d'une phase de nitrobenzène non miscible formée, contenant le catalyseur d'hydrogènation, d'avec une phase aqueuse formée contenant le sulfate de produite (PAP), d'autres corps destinés à réagir et qui n'ont pas servi et des sous-produits;

la neutralisation, à l'aide d'une base efficace, de la phase aqueuse contenant le sulfate de PAP;

la dissolution et l'isolement du PAP formé dans un solvant organique basique, non miscible à l'eau, et le transport de la solution du PAP dans le solvant vers une zone d'amination;

l'amination de la solution de solvant contenant le PAP, à l'aide d'un excès molaire d'ammoniac, pour convertir le PAP qui y est contenu en du PPD, à des températures comprises entre 200°C et 400°C et à des pressions comprises entre 345 et 6890 kPa (pression absolue de 50 à 1000 livres par pouce carré);

la séparation du PPD formé d'avec le mélange des sous-produits de la réaction dans le solvant organique basique.

2. Procédé tel que revendiqué à la revendication 1, dans lequel le catalyseur d'hydrogénation est choisi parmi du platine sur du carbone et du palladium sur du carbone.

3. Procédé tel que revendiqué à la revendication 1 ou à la revendication 2, dans lequel on recycle vers la zone de réduction la phase de nitrobenzène non miscible, n'ayant pas réagi et contenant le catalyseur d'hydrogénation.

4. Procédé tel que revendiqué dans l'une quelconque des revendications précédentes, dans lequel la base efficace neutralisant la phase aqueuse contenant du sulfate de PAP est de l'ammoniac ou de l'ammoniaque.

5. Procédé tel que revendiqué dans l'une quelconque des revendications précédentes, dans lequel on met en contact et l'on mélange, dans une zone d'extraction, la solution contenant du PAP avec le solvant organique basique non miscible à l'eau.

6. Procédé tel que revendiqué à la revendication 5, dans lequel on sépare le mélange résultant en un premier courant comprenant le solvant transportant le PAP dissous et en un second courant transportant divers autres produits de réaction et un peu de solvant.

7. Procédé tel que revendiqué dans l'une quelconque des revendications précédentes, dans lequel le solvant organique basique non miscible à l'eau est choisi parmi l'aniline, la quinoléine, le benzonitrile, la toluidine, la xylidine, la picoline, la lutidine, la quinaldine et leurs mélanges.

8. Procédé tel que revendiqué à la revendication 7, dans lequel le solvant est l'aniline.

9. Procédé tel que revendiqué dans l'une quelconque des revendications précédentes, dans lequel on maintient la zone d'amination à des températures de 300 à 380°C et à des pressions de 345—3450 kPa (pression absolue de 50 à 500 livres par pouce carré).

10. Procédé tel que revendiqué dans l'une quelconque des revendications précédentes, dans lequel la quantité de PAP dans la zone d'amination se situe entre 10 et 50% du poids du solvant présent.

11. Procédé tel que revendiqué dans l'une quelconque des revendications précédentes, dans lequel on effectue d'amination en présence d'un catalyseur à base d'alumine acide.

12. Procédé tel que revendiqué dans l'une quelconque des revendications précédentes, dans lequel on enlève du mélange réactionnel, dans une zone de séparation par cristallisation, le PPD produit.

13. Procédé tel que revendiqué dans l'une quelconque des revendications précédentes, dans lequel on conduit le procédé en continu.

14. Procédé tel que revendiqué dans l'une quelconque des revendications précédentes, dans lequel on recycle le solvant pour le mettre en contact, dans la zone d'extraction, avec une solution fraîche contenant du PAP.

FIG. 1